(19) Europäisches Patentamt
European Patent Office
Office européen des brevets

(11) Numéro de publication: **0 251 953 A1**

(12) # DEMANDE DE BREVET EUROPEEN

(21) Numéro de dépôt: **87420185.8**

(22) Date de dépôt: **01.07.87**

(51) Int. Cl.[4]: **C 07 C 79/10**
C 07 C 76/02

(30) Priorité: **04.07.86 FR 8609948**

(43) Date de publication de la demande:
**07.01.88 Bulletin 88/01**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Desbois, Michel**
**526, Chemin du Bois**
**F-69140 Rilleux La Pape (FR)**

(74) Mandataire: **Cazes, Jean-Marie et al**
**RHONE-POULENC INTERSERVICES Service Brevets Chimie 25, quai Paul Doumer**
**F-92408 Courbevoie Cédex (FR)**

(54) **Procédé de préparation de dinitroalkylbenzènes.**

(57) La présente invention concerne un procédé de préparation de dinitroalkylbenzène où l'on met en contact au sein de l'acide fluorhydrique liquide un alkylbenzène et de l'acide nitrique.

EP 0 251 953 A1

**Description**

## PROCEDE DE PREPARATION DE DINITROALKYLBENZENES

La présente invention concerne un procédé de préparation de dinitroalkylbenzènes. Elle concerne plus particulièrement un procédé de préparation de dinitrotoluène.

Les procédés de dinitration de composés benzéniques connus jusqu'à ce jour sont peu nombreux. Il est connu d'après l'article de Mary L. KILPATRICK et de Martin KILPATRICK (J. Phys. Chem. 69 (7), 2248-55 -1965-) de nitrer un nitrobenzène à l'aide de tétrafluoroborate de nitronium dans l'acide fluorhydrique. La conclusion de cet article est que la nitration est fortement ralentie en milieu acide fluorhydrique par rapport à la même réaction réalisée en milieu sulfurique. D'autre part l'emploi de tétrafluoroborate de nitronium ne peut être extrapolé au niveau industriel car c'est un produit de laboratoire non disponible en grande quantité et de toute façon d'un prix trop élevé.

D'autres articles décrivent l'utilisation de l'acide nitrique ou d'un sel alcalin de l'acide nitrique en milieu fluorhydrique comme agent de nitration tel que le brevet US 3 326 983 ou l'article de SIMONS (J. Am. Chem. Soc. 63, 608-9 -1941-). D'après ces articles on n'obtient aucun dérivé dinitré.

En présence d'acide fluorhydrique seul le nitrobenzène ne subit aucune nitration supplémentaire. En présence d'acide fluorhydrique et de fluorure de sodium nous pouvons confirmer, d'après nos propres expérimentations qu'il n'apparait qu'une faible quantité de dérivés dinitrés (environ 10%).

La présence de fluorure de sodium permet essentiellement d'inverser le rapport isomérique orthonitrotoluène par rapport au paranitrotoluène.

Aucun procédé ne décrit de procédés d'obtention simple et économique de dinitroalkylbenzène et plus particulièrement de dinitrotoluène.

La présente invention permet d'atteindre cet objectif et a pour objet un procédé de préparation de dinitroalkylbenzène par mise en contact en milieu acide fluorhydrique d'un alkylbenzène et d'acide nitrique.

Contrairement à tout ce qui est décrit dans l'art antérieur l'acide nitrique en présence d'acide fluorhydrique permet de dinitrer tout dérivé benzénique substitué par un groupe alkyle.

Les alkylbenzènes mis en oeuvre dans la présente invention répondent à la formule générale suivante :

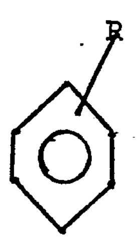

(I)

dans laquelle R représente un groupe alkyle ayant de l à 4 atomes de carbone et de préférence un groupe méthyle.

L'acide nitrique utilisé est un acide courant du commerce tel que notamment l'acide nitrique à 70 % ou à 98 %.

L'acide fluorhydrique utilisé est de préférence anhydre bien qu'un acide fluorhydrique contenant de l'eau ne soit pas à exclure.

L'eau ralentit seulement la réaction ; comme une partie de l'eau est déjà apportée par l'acide nitrique commercial qui n'est jamais anhydre, il est préférable, afin de ne pas trop ralentir la réaction, de travailler avec de l'acid fluorhydrique anhydre.

On peut travailler en présence de tout solvant inerte tel que notamment le chlorure de méthylène, le chloroforme, le tétrachlorure de carbone, le trifluoro-1,1,2 trichloro-1,2,2 éthane et le trichloro 1,1,1 éthane.

Pour une meilleure mise en oeuvre de la réaction on préfère opérer en présence d'une quantité d'acide nitrique telle que le rapport acide nitrique à l'alkylbenzène soit supérieur à 2 et de préférence compris entre 2 et 2,5.

L'acide fluorhydrique étant utilisé comme solvant, la quantité maximale sera simplement adaptée par l'homme de l'art à l'économie du procédé. Pour une mise en oeuvre optimale on choisira une quantité d'acide fluorhydrique telle que le rapport molaire acide fluorhydrique à l'alkylbenzène soit compris entre 5 et 50 et de préférence entre 10 et 30.

Pour une meilleure mise en oeuvre de l'invention on préfère opérer à une température comprise entre -20 et 100°C et de préférence entre 0 et 50°C.

La pression réactionnelle n'est pas un critère essentiel du procédé. Néanmoins lorsque la température dépasse 20°C on préfère opérer sous une pression supérieure à la pression atmosphérique de façon à conserver l'acide fluorhydrique sous forme liquide.

Les produits issus du procédé de l'invention que sont les nitroalkylbenzènes et de préférence le dinitrotoluène sont extraits du milieu réactionnel à l'aide d'un solvant tel que le chlorure de méthylène ou par distillation de l'acide fluorhydrique. Ils sont utilisés notamment comme intermédiaires dans la fabrication des mousses de polyuréthanne.

L'invention va être plus complètement décrite à l'aide des exemples suivants qui ne doivent pas être considérés comme limitatifs de l'invention.

## EXEMPLES

### Exemple 1 : essai sans solvant

Dans un réacteur inox de 250 ml agité par barreau aimanté. On introduit successivement 100 g (5 moles) d'acid fluorhydrique anhydre, 9,2 g (0,1 mole) de toluène et 14,5 g (0,22 mole) d'acide nitrique 98 %. Le réacteur est fermé puis maintenu sous agitation à la température de 15°C pendant 1 h 20. En fin de réaction, le brut réactionnel acide est coulé sur 200 g de glace pilée, extrait par 3 x 100 $cm^3$ de $CH_2Cl_2$, les phases organiques rassemblées et lavées par 2 x 100 $cm^3$ d'eau, enfin séchées. Après évaporation, on récupère 18 g d'un mélange constitué de (Analyse chromatographie gazeuse capillaire - Normalisation à 100) :
- 2,6 dinitrotoluène : 19,7 %
- 2,3 et 2,5 dinitrotoluène : 0,54 %
- 2,4 dinitrotoluène : 75,5 %
- 3,4 dinitrotoluène : 2,3 %
- inconnus : 1,92 %

Exemple 2 : essai avec solvant ($CH_2Cl_2$ )

Dans un réacteur inox de 250 ml agité par barreau aimanté, on introduit successivement 30 g (1,5 mole d'acide fluorhydrique anhydre, 18,4 g (0,2 mole) de toluène, 27,7 g (0,42 mole) d'acide nitrique 98 % et132 g de chlorure de méthlène. Le réacteur est fermé puis maintenu sous agitation à la température de 20°C pendant 2 h 50. En fin de réaction, on récupère par décantation une phase supérieure acide de 20 $cm^3$ et une phase inférieure organique de 126 m3. Cette dernière est coulée sur 60 g de glace pilée puis décantée. La phase organique ainsi obtenue est lavée par 2 x 100 $cm^3$ d'eau séchée et évaporée. On recueille ainsi 29 g d'un mélange constitué de (Analyse chromatographie phase gazeuse ) Normalisation à 100) :
- Toluène : 0,01 %
- Mononitrotoluène : 28,3 %
- 2,6 dinitrotoluène : 16,6 %
- 2,4 dinitrotoluène : 53,5 %
- 2,3 et 2,5 dinitrotoluène : 0,25 %
- 3,4 dinitrotoluène : 1,1 %

**Revendications**

1. Procédé de préparation de dinitroalkylbenzène caractérisé en ce que l'on met en contact au sein de l'acide fluorhydrique liquide un alkylbenzène et de l'acide nitrique.

2. Procédé selon la revendication 1 caractérisé en ce que l'alkylbenzène répond à la formule suivante (I)

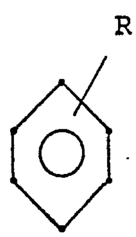

(I)

dans laquelle R représente un groupe alkyle ayant 1 à 4 atomes de carbone et de préférence un groupe méthyle.

3. Procédé selon la revendication 1 caractérisé en ce que l'acide nitrique est un acide nitrique à 70 % ou 98 %.

4. Procédé selon la revendication 1 caractérisé en ce que l'acide fluorhydrique est anhydre.

5. Procédé selon la revendication 1 caractérisé en ce que l'on met en contact l'acide l'acide fluorhydrique, l'alkylbenzène et l'acide nitrique en présence d'un solvant choisi parmi le chlorure de méthylène, le chloroforme, le tétrachlorure de carbone, le trifluoro-1,1,2 trichloro-1,2,2 éthane et le trichloro-1,1,1 éthane.

6. Procédé selon la revendication 1 caractérisé en ce que le rapport molaire de l'acide nitrique à l'alkylbenzène est supérieur à 2 et de préférence compris entre 2 et 2,5.

7. Procédé selon la revendication 1 caractérisé en ce que le rapport molaire de l'acide fluorhydrique à l'alkylbenzène est compris entre 5 et 50 et de préférence entre 10 et 30.

8. Procédé selon la revendication 1 caractérisé en ce que la température de réaction est comprise entre -20 et 100°C et de préférence entre 0 et 50°C.

9. Procédé selon la revendication 1 caractérisé en ce que la pression réactionnelle est supérieure à 1 bar.

Office européen des brevets

# RAPPORT DE RECHERCHE EUROPEENNE

Numéro de la demande

EP 87 42 0185

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | US-A-3 326 983 (J.A. VESELY)<br>* Revendications; exemple III * | 1-9 | C 07 C 79/10<br>C 07 C 76/02 |
| A | US-A-3 417 146 (C.B. LINN)<br>* Revendications; exemple III * | 1-9 | |
| A | DE-C- 529 538 (K. FREDENHAGEN)<br>* En entier * | 1-9 | |
| A,D | JOURNAL OF THE AMERICAN CHEMICAL SOCIETY, vol. 63, no. 2, 6 février 1941, pages 608-609; L.F. FIESER et al.: "Synthesis of 10-methyl-3'-isopropyl-1,2-benzanthracene from 9,10-dihydroretene"<br>* En entier * | 1-9 | |

DOMAINES TECHNIQUES RECHERCHES (Int. Cl.4)

C 07 B 43/00
C 07 C 76/00
C 07 C 79/00

Le présent rapport de recherche a été établi pour toutes les revendications

| Lieu de la recherche | Date d'achèvement de la recherche | Examinateur |
|---|---|---|
| LA HAYE | 09-10-1987 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant